# EUROPÄISCHE PATENTSCHRIFT

(11) **EP 1 496 043 B1**
(45) Veröffentlichungstag und Bekanntmachung des Hinweises auf die Patenterteilung: **31.03.2010**
(21) Anmeldenummer: 04014227.5
(22) Anmeldetag: 17.06.2004
(51) Int. Cl.: C07C 201/16, C07C 205/06, C02F 1/26

(54) **Verfahren zur Aufarbeitung des bei der Herstellung von Dinitroluol anfallenden Abwassers**
Process for treating the waste water of the dinitrotoluene production process
Procédé de traitement des eaux usées du procédé de fabrication de dinitrotoluène

(30) Priorität: 30.06.2003 DE 10329304
(43) Veröffentlichungstag der Anmeldung: 12.01.2005
(73) Patentinhaber: Bayer MaterialScience AG, 51368 Leverkusen (DE)
(72) Erfinder: Münnig, Jürgen, 41564 Kaarst (DE); Wastian, Dietmar, 41542 Dormagen (DE); Lorenz, Wolfgang, Dr., 41540 Dormagen (DE); Keggenhoff, Berthold, Dr., 47839 Krefeld (DE)

(56) Entgegenhaltungen:
- US-A- 3 742 072
- US-B1- 6 254 789
- US-B1- 6 506 948

## Beschreibung

Gegenstand der Erfindung ist ein Verfahren zur Aufarbeitung des bei der Herstellung von Dinitrotoluol (DNT) durch Nitrierung von Toluol anfallenden Reaktions- und Waschwassers. Eine Behandlung des Reaktions- und Waschwassers ist erforderlich um DNT-Verluste zu vermeiden und um das Prozessabwasser einer biologischen Aufarbeitung zuführen zu können.

Bei den üblichen Verfahren zur Herstellung von Dinitrotoluol (DNT) aus Toluol und einem Gemisch aus Schwefel- und Salpetersäure (Nitriersäure) fallen das bei der Schwefelsäureaufkonzentrierung abdestillierte saure Reaktionswasser sowie alkalisches und saures Waschwasser aus der Reinigung des DNT als wässrige Abwässer an. Diese wässrigen Phasen enthalten Monound Dinitrotoluol sowie Nebenprodukte der Nitrierung wie zum Beispiel Mono-, Di- und Trinitrokresole (im Folgenden unter dem Begriff Nitrokresole zusammengefasst), Pikrinsäure und Nitrobenzoesäuren. Die Entfernung dieser Stoffe aus den Wasserphasen ist aus zwei Gründen erforderlich:
1. Mit Konzentrationen bis über 2,5 Gew.-% DNT und 1,5 Gew.-% MNT im Prozesswasser stellt die Entsorgung des unbehandelten Abwassers einen Ausbeuteverlust dar.
2. Aromatische Nitroverbindungen sind in biologischen Kläranlagen schwer abbaubar und weisen bakterientoxische Eigenschaften auf.

In bisherigen Arbeiten über die Nitrierung aromatischer Verbindungen mit Behandlung der entstehenden wässrigen Phasen werden verschiedene. Verfahren beschrieben.

US-A-6,506,948 behandelt die Nitrierung von Toluol wobei die anfallenden wässrigen Phasen jeweils direkt mit Toluol extrahiert werden. Eine Organika-Abscheidung vor der Extraktion ist gemäß US-A-6,506,948 nicht vorgesehen. Bei der Extraktion werden die wässrigen sauren Phasen aus der Schwefelsäureaufkonzentrierung, sowie eine wässrige saure Phase und eine wässrige alkalische Phase aus der DNT-Wäsche mit Toluol extrahiert, wobei der Toluolstrom in Reihe nacheinander den einzelnen wässrigen Phasen zugeführt wird. Anschließend wird der Toluolstrom im Nitrierprozess verarbeitet. Somit werden die verschiedenen Abwässer separat mit Toluol behandelt, was für jeden Abwasserstrom eine eigene Mischer-Scheider-Apparatur erfordert.

Im US-A-4,642,396 wird die Überführung von Nitrierprodukten aus der wässrigen Säurephase in die organische Phase im Nitriergemisch durch Einleiten von Stickstoffmonoxid beschrieben. Dabei wird noch vorhandene Salpetersäure zu Stickstoffdioxid reduziert, welches in einem geeigneten nachgeschalteten Prozess in Salpetersäure umgewandelt werden kann. Allerdings ist dabei der Einsatz und die Verwertung des prozessfremden Stoffs Stickstoffdioxid erforderlich, die offensichtlich in einer weiteren Anlage zur Herstellung von Salpetersäure erfolgt.

Die Rückgewinnung von Nitroaromaten (Nitrobenzol) aus alkalischem Waschwasser durch Extraktion mit dem Edukt des Nitrierprozesses (Benzol) wird in US-A-4,241,229 für die Nitrierung von Benzol zu Nitrobenzol nach dem Nitriersäureverfahren beschrieben. Darin wird die Abtrennung von Nitroaromaten aus Abwasser und Waschwasser durch Dampfstrippung erwähnt. Diese Arbeit beschreibt zwar die Rückgewinnung von Produkt, jedoch wird eine weitere Behandlung der im alkalischen Waschwasser gelösten Salze von Phenolen, Pikrinsäure und organischen Säuren nicht beschrieben.

Die Behandlung von Nitrokresolen, die vom Produktstrom mittels alkalischer DNT-Wäsche abgetrennt werden, durch oxidativen Abbau mit Salpetersäure bei erhöhten Temperaturen erfordert eine eigene separate Verfahrenstufe wie in EP-A1-0 962 446 beschrieben.

In US-A-4,230,567 wird im Rahmen der Nitrobenzolherstellung ebenfalls ein Nitrophenolabbau in einem eigenen zusätzlichen Verfahrensschritt bei erhöhtem Druck und Temperatur beschrieben.

Die Abtrennung von Nitrokresolkomponenten aus dem Produkt Dinitrotoluol und dem anfallenden Waschwasser wird in US-A-4,597,875 folgendermaßen vorgeschlagen: Nitrokresole werden mit alkalischem Waschwasser aus der DNT-Phase in einem Extraktiönsschritt als wasserlösliche Salze entfernt. Das alkalische Waschwasser wird nach Abtrennung von DNT mit Schwefel- oder Salpetersäure zur Fällung der Nitrokresolkomponenten versetzt. Nach mechanischer Abscheidung der Nitrokresolphase kann diese in einem geeigneten Verbrennungsprozess entsorgt werden. Hier werden die Einsatzstoffe Salpetersäure und Schwefelsäure zur Abtrennung der Nebenkomponenten verbraucht. Zusätzlich wird eine Fremdentsorgung der Nitrokresolkomponenten erforderlich.

Gegenstand der Patentschrift US-A-4,257,986 ist die Behandlung von in der Nitrierung aromatischer Verbindungen mit Nitriersäure anfallender Schwefelsäure. Zur Entfernung von Salpetersäure, salpetriger Säure und organischen Verunreinigungen in dieser Schwefelsäure wird diese mit dem zu nitrierenden Aromaten (Edukt) sowie mit verschiedenen Reduktions- bzw. Oxidationsmitteln behandelt. Auch dieses Verfahren macht den Einsatz zusätzlicher prozessfremder Stoffe erforderlich.

Aufgabe der vorliegenden Erfindung ist daher, ein Verfahren zur Rückgewinnung der in den verschiedenen wässrigen Abwässern enthaltenen Nitrierprodukte sowie zur Abtrennung und Behandlung unerwünschter Nebenkomponenten der Nitrierung zur Verfügung zu stellen, das einfach und wirtschaftlich ist. Überraschenderweise gelingt diese Aufgabe im Vergleich zu bisherigen Prozessen mit verfahrenstechnisch einfachen Schritten ohne den Einsatz zusätzlicher prozessfremder Verfahrensschritte und Einsatzstoffe.

Die Erfindung betrifft ein Verfahren zur Aufarbeitung von wässrigen Abwässern, die bei der Nitrierung von Toluol zu Dinitrotoluol mit Nitriersäure anfallen, wobei die wässrigen Abwässer saures Waschwasser und alkalisches Waschwasser aus der Wäsche von Dinitrotoluol und Destillat aus der Schwefelsäureaufkonzentrierung enthalten, bei dem
a) die sauren und alkalischen Abwässer aus der Wäsche und das wässrige Destillat aus der Schwefelsäureaufkonzentrierung vereinigt werden, so dass sich ein pH-Wert von unter 5 (gemessen bei 70°C) einstellt, und anschließend die entstehende wässrige und organische Phase durch Phasentrennung getrennt werden, und
b) die wässrige Phase aus Schritt a) einer Extraktion zugeführt wird, wobei die in der wässrigen Phase enthaltenen organischen Komponenten mit Toluol extrahiert und die mit den organischen Komponenten angereicherte Toluolphase der Nitrierung von Toluol zugeführt wird.

Im üblichen Nitrierverfahren aromatischer Kohlenwasserstoffe wird der Kohlenwasserstoff mit einem Gemisch aus Schwefelsäure und Salpetersäure (Nitriersäure) zur Reaktion gebracht. Im Fall der Nitrierung von Toluol zu Dinitrotoluol ist neben dem einstufigen Prozess (EP-A2-908 442) eine zweistufige Nitrierung das allgemein gängige Verfahren. Im. zweistufigen Verfahren wird zunächst Toluol mit Salpetersäure und Schwefelsäure zum Mononitrotoluol (MNT) umgesetzt (Monostufe). Nach Trennung des entstandenen Reaktionsgemisches in MNT und eine Säurephase, was in statischen Abscheidern oder in dynamischen Abscheidern ausgeführt werden kann, wird das MNT erneut mit Salpetersäure und Schwefelsäure zum Dinitrotoluol (DNT) umgesetzt (Di-Stufe). Die Schwefelsäurephase aus der Monostufe wird aufkonzentriert. In der Di-Stufe wird aufkonzentrierte Schwefelsäure eingesetzt. Das Reaktionsgemisch der Di-Stufe wird in eine organische Phase, das Roh-DNT, und eine Säurephase getrennt, wobei die Säurephase als Einsatz-Schwefelsäure der Mono-Stufe dienen kann oder einer Aufkonzentrierung zugeführt wird. Die Trennung dieses Reaktionsgemisches der Di-Stufe kann ebenfalls in statischen oder dynamischen Abscheidern erfolgen.

In allen Herstellverfahren von DNT durch Toluolnitrierung mit Nitriersäure fallen zwei Stoffströme an, die einer weiteren Aufarbeitung zugeführt werden müssen, das Roh-DNT und die durch Reaktionswasser und den Wasseranteil der eingesetzten Salpetersäure verdünnte Schwefelsäure.

Das Roh-DNT besteht im Allgemeinen im wesentlichen aus dem gewünschten Reaktionsprodukt mit bis zu 1,5 Gew.-% Schwefelsäure sowie überschüssiger Salpetersäure von 0,5 Gew.-% bis 1,2 Gew.-% und Nebenprodukten der Nitrierung in einer Konzentration von bis zu ca. 1 Gew.-%. Die Nebenkomponenten sind im wesentlichen Nitrokresole, Pikrinsäure und Nitrobenzoesäuren. Üblicherweise wird das Roh-DNT in zwei bis vier Waschstufen mit Wasser von Säuren und Nebenkomponenten befreit. Das in diesem Prozess zugeführte Waschwasser kann in mindestens einer Waschstufe eine Base enthalten, wobei üblicherweise Natronlauge oder Natriumkarbonat in Konzentrationen von 2 - 10 Gew.-% eingesetzt wird. Während durch die neutrale Wasserwäsche weitgehend Schwefelsäure und Salpetersäure aus dem Nitrierprodukt entfernt werden, überführt die alkalische Wäsche auch salzbildende organische Komponenten wie zum Beispiel Nitrokresole, Pikrinsäure und Nitrobenzoesäuren in die wässrige Phase.

Als Waschwasser kann, außer für die einstufige alkalische Wäsche sowie die letzte Wasserwäsche, jeweils Frischwasser oder im Gegenstrom geführtes Waschwasser einer Folgestufe eingesetzt werden. Das eingesetzte Waschwasser kann aber auch Frischwasser, demineralisiertes Wasser oder Wasser einer geeigneten Qualität aus einem Folgeprozess des beschriebenen Nitrierverfahrens sein.

Die eingesetzten Mengen an Waschwasser betragen bevorzugt 15 - 90 Gew.-Teile, besonders bevorzugt 50 - 65 Gew.-Teile Waschwasser pro 100 Gew.-Teile DNT.

In der neutralen Wasserwäsche entsteht je nach eingesetzter Waschwassermenge und Waschwasserführung ein saures Prozessabwasser mit bevorzugten Säuregehalten von 1,0 bis 3,0 Gew.-% Salpetersäure und 2,0 - 6,0 Gew.-% Schwefelsäure sowie einem DNT Gehalt von einigen 1000 ppm. Die Konzentration an organischen Nebenprodukten der Nitrierung liegt im genannten Prozessabwasser im Allgemeinen zwischen 300 und 900 ppm.

Im Abwasserstrom der alkalischen Wäsche sind im Allgemeinen 3,0 bis 7,0 Gew.-% organische Nebenprodukte der Nitrierung, im wesentlichen Nitrokresole, Pikrinsäure und Nitrobenzoesäuren, in Form ihrer wasserlöslichen Salze der eingesetzten Base enthalten. Daneben kann dieser Abwasserstrom auch noch mehrere 1000 ppm DNT sowie 2,0 - 4,0 Gew.-% Salpetersäure und ca. 0,6 - 1,2 Gew.-% Schwefelsäure in Form ihrer wasserlöslichen Salze enthalten. Der Abwasserstrom der alkalischen Wäsche hat einen pH-Wert > 7,0, vorzugsweise >7,5 (gemessen bei 80°C).

Die Waschstufen werden in geeigneten Apparaten, vorzugsweise in Wasch- bzw. Extraktionskolonnen oder in Mischer-Scheider Apparaturen durchgeführt.

Die verdünnte Schwefelsäure aus der Nitrierung kann zu 70 - 90 Gew.-%, bevorzugt zu 70 - 80 Gew.-%, besonders bevorzugt zu 75 - 79 Gew.-%, aus Schwefelsäure bestehen und kann noch 0,005 - 0,5 Gew.-%, vorzugsweise 0,005 - 0,05 Gew.-% Salpetersäure sowie bis zu 3,0 Gew.-% MNT und 0,2 - 2,0 Gew.-% DNT enthalten. Daneben enthält die aufzuarbeitende Säure noch bis zu 0,2 Gew.-% organische Nebenkomponenten, die im wesentlichen aus Nitrokresolen, Pikrinsäure und Nitrobenzoesäuren bestehen. Für die Aufkonzentrierung der verdünnten Schwefelsäure stehen neben anderen möglichen Verfahren zum Beispiel das Pauling-Verfahren bei Normaldruck [Bodenbrenner, von Plessen, Vollmüller, Dechema-Monogr. 86 (1980), 197] mit einer ca. 97 %igen Schwefelsäure als Produkt, sowie die Vakuumeindampfung [DE-A1-196 42 328], die ebenfalls eine Schwefelsäure bis 97 % liefern kann, zur Verfügung. Neben der gewünschten Schwefelsäure erhält man nach Kondensation der Brüden im Allgemeinen eine oder mehrere wässrige Phasen mit einem Schwefelsäureanteil von 0,2 - 1,0 Gew.-%, vorzugsweise 0,2 - 0,6 Gew.-%, und Gehalten an MNT von 0,7 - 7,0 Gew.-% sowie DNT von 2,0 - 6,0 Gew.-%. Weitere organische Verbindungen sind üblicherweise in Konzentrationen von bis zu 0,4 Gew.-% enthalten. Die organischen Anteile im Destillat sind gelöst bzw. dispergiert.

Im erfindungsgemäßen Verfahren werden die Abwasserströme der neutralen und der alkalischen DNT-Wäsche, und der Schwefelsäureaufkonzentrierung vereint. Die wässrigen Phasen aus der Nitrierung setzen sich dabei aus mehreren, vorzugsweise zwei bis vier Einzelströmen zusammen, wobei mindestens einer der Einzelströme aus der neutralen Wasserwäsche stammt (saures Waschwasser) und mindestens ein Einzelstrom aus der alkalischen Wäsche stammt (alkalisches Waschwasser). Die Wasserphasen aus der Schwefelsäureaufkonzentrierung bestehen aus einem oder mehreren Einzelströmen mit genannten Anteilen an Säuren und organischen Komponenten.

Die Vereinigung der Prozessabwasserströme kann in einem dafür geeigneten Behälter mit dynamischem Mischelement oder mittels einer statischen Mischeinheit erfolgen. Nach der Zusammenführung der genannten Ströme stellt sich ein pH-Wert der Mischung unterhalb von 5 (gemessen bei 70°C), vorzugsweise unterhalb von 2 ein, wobei sich eine organische Phase abscheidet. Wenn die alkalische Wäsche mit sehr großen Mengen an Base durchgeführt wird, dann ist es theoretisch möglich, dass bei der Vereinigung der Abwässer aus der DNT-Wäsche und des Destillats aus der Schwefelsäureaufkonzentrierung ein pH-Wert ≥ 5 erhalten wird. Dann müsste zum Beispiel die Menge des eingesetzten alkalischen Abwassers entsprechend reduziert werden, um einen pH-Wert von kleiner 5 zu erreichen. Diese organische Phase besteht aus MNT und DNT sowie Nebenprodukten der Nitrierung, vorwiegend Nitrokresolen, Pikrinsäure und Nitrobenzoesäuren. Im Falle der Benutzung von Karbonat in der alkalischen Wäsche muss eine entsprechende Entgasungsmöglichkeit beim Zusammenführen der Ströme vorhanden sein. Zur Abtrennung der gebildeten organischen Phase werden die vereinigten Abwasserströme anschließend in ein geeignetes Scheidegefäß geleitet.

Der Vorteil des erfindungsgemäßen Verfahrens besteht darin, dass durch die Vereinigung der verschiedenen sauren und alkalischen Prozessabwasserströme Nebenprodukte der Nitrierung, vorwiegend Nitrokresole, Pikrinsäure und Nitrobenzoesäuren, als getrennte organische Phase abgeschieden werden können, ohne dass dazu zusätzlich eine der Einsatzsäuren des Nitrierprozesses verbraucht wird. Zusätzlich ist nach der Zusammenführung dieser Prozessabwässer lediglich ein einziger Abwasserstrom einer weiteren Aufarbeitung zu unterziehen.

Bei der Vereinigung der Prozessabwasserströme kann zusätzlich MNT eingespeist werden. Eine MNT-Zugabe kann die Phasentrennung unterstützen und durch Absenkung des Festpunktes der organischen Phase die Förderung dieses Stoffgemisches im Prozess erleichtern. Die eingesetzten Mengen betragen bevorzugt 0,2 - 9,0 Gew.-Teile, besonders bevorzugt 0,5 - 4,0 Gew.-Teile MNT pro 100 Gew.-Teile Prozessabwasser.

Aufgrund der Dichteunterschiede bildet die organische Phase, vorwiegend bestehend aus MNT, DNT, Nitrokresolen, Pikrinsäure und Nitrobenzoesäuren, üblicherweise die schwerere Phase. Diese organische Phase kann aus dem Scheidegefäß entnommen und einer Entsorgung, z.B. Verbrennung, zugeführt werden. Bevorzugt werden diese Organika aber dem Nitrierprozess wieder zugeführt.

Die wässrige Phase aus dem Abscheider wird einer Abwasserextraktion zugeführt. In dieser wässrigen Phase findet man im Allgemeinen organische Komponenten in folgenden Konzentrationsbereichen: MNT von 50 bis 1000 ppm, DNT von 100 bis 3000 ppm und Nitrokresole, Pikrinsäure und Nitrobenzoesäuren von 100 bis 3000 ppm in der Summe. Daneben sind noch Schwefel- und Salpetersäure in Konzentrationen von jeweils 0,4 bis 2,0 Gew.-% enthalten.

Die Extraktion dieser wässrigen Phase wird mit Toluol, dem Edukt der Nitrierung, durchgeführt. Die Extraktion erfolgt in dafür geeigneten Mischer/Scheider-Einheiten oder gepulsten Packungsund Siebbodenkolonnen, vorzugsweise aber in gerührten, mehrstufigen Extraktionskolonnen, wobei Toluol und wässrige Phase im Gegenstrom gefahren werden. Diese Extraktion kann einstraßig oder mehrstraßig mit mehreren parallel laufenden Extraktionskolonnen betrieben werden. Die Zugabe des Extraktionsmittels kann direkt in die Extraktionskolonne erfolgen. Zusätzlich kann Toluol über eine statische Mischeinheit in die wässrige Phase vor Zugabe in den Extraktionsapparat geführt werden. Das Gewichtsverhältnis von eingesetztem Toluol zu eingesetzter wässriger Phase kann im Bereich von 33:100 bis 5:100, vorzugsweise 33:100 bis 10:100 liegen. Durch dieses Extraktionsverfahren kann die Konzentration der Nitrierprodukte und organischen Nebenprodukte in der wässrigen Phase überraschenderweise unter 10 ppm MNT und DNT und unter 1000 ppm, bevorzugt unter 200 ppm Nitrokresole, Pikrinsäure und Nitrobenzoesäuren abgereichert werden, wobei üblicherweise ca. 500 - 2000 ppm Toluol in die wässrige Phase aufgenommen werden.

Der Toluolstrom aus der Extraktion wird dann dem Nitrierprozess als Rohstoff zugeführt.

Die wässrige Phase, die aus der Extraktion erhalten wird, enthält üblicherweise ca. 500 bis 2000 ppm an Toluol. Dieses Toluol kann mittels Dampfstrippung aus der wässrigen Phase entfernt werden. Dazu wird die wässrige Phase auf den Kopf einer Strippkolonne aufgegeben. Die Kolonne wird bevorzugt bei einem Druck von 200 - 400 mbar, besonders bevorzugt bei 200 - 300 mbar betrieben. Im unteren Teil der Kolonne wird Dampf einer geeigneten Druckstufe, vorzugsweise 2-6 bar aufgegeben. Zwischen dem Ort der Aufgabe der wässrigen Phase und der Dampfeinspeisung ist die Kolonne bevorzugt mit Packung, Füllkörper oder Böden bestückt. Überraschenderweise enthält das so behandelte Abwasser Toluolkonzentrationen von unter 10 ppm.

Das mit der wässrigen Phase in die Dampfstrippung eingebrachte Toluol verdampft in der Strippkolonne und wird zusammen mit Wasserdampf einer Kondensation zugeführt. Das auf diese Art erhaltene Toluol / Wasser-Gemisch kann neben anderen Einsatzmöglichkeiten zur Abwasserextraktion zurückgeführt oder einer Phasentrennung unterzogen werden, wonach das abgeschiedene Toluol dann der Nitrierung zugeführt werden kann.

In den folgenden Beispielen wird das erfindungsgemäße Verfahren näher erläutert:

### Beispiele

### Beispiel 1

Vereinigung der Prozessabwasserströme mit Phasentrennung und Abscheidung der wässrigen von der organischen Phase
62,07 kg/h Prozesswasser aus der Schwefelsäureaufkonzentrierung (Strom A), 22,08 kg/h saures Waschwasser (Strom B) und 6,13 kg/h alkalisches Waschwasser (Strom C) aus der DNT-Wäsche mit einer durchschnittlichen Temperatur von 70°C werden über eine gemeinsame Abwassersammelleitung und einen statischen Inline-Waschwassermixer in einen Organika-Abscheider geleitet. Dieser Abscheider besteht aus einem liegenden Behälter von 100 mm Durchmesser und einer Länge von 600 mm. Der Behälter ist mit einem internen Überlaufwehr ausgestattet. Aus dem kontinuierlich betriebenen Abscheidergefäß werden im Überlauf als leichtere Phase 86,73 kg/h von ungelösten organischen Anteilen befreites Prozessabwasser (Strom D) entnommen. Die abgetrennten Organika (Strom E) stellen die schwerere Phase dar und werden über eine Bodenentnahme aus dem Abscheider entnommen.

**Tabelle 1**

| Komponente | Strom A | Strom B | Strom C | Strom D | Strom E |
|---|---|---|---|---|---|
| | [kg/h] | [kg/h] | [kg/h] | [kg/h] | [kg/h] |
| MNT | 1,10 | 0,00 | 0,00 | 0,01 | 1,09 |
| DNT | 1,76 | 0,18 | 0,05 | 0,03 | 1,96 |
| NITROKRESOLE, PIKRINSÄURE, NITROBENZOESÄUREN | 0,22 | 0,01 | 0,31 | 0,05 | 0,49 |
| HNO3 | 0,18 | 0,24 | 0,00 | 0,40 | 0,00 |
| HNO2 | 0,05 | 0,03 | 0,00 | 0,08 | 0,00 |
| H2SO4 | 0,13 | 0,61 | 0,00 | 0,73 | 0,00 |
| H2O | 58,63 | 20,97 | 5,43 | 85,03 | 0,00 |
| NA2CO3 | 0,00 | 0,00 | 0,02 | 0,00 | 0,00 |
| NA2SO4 | 0,00 | 0,00 | 0,08 | 0,09 | 0,00 |
| NANO3 | 0,00 | 0,00 | 0,23 | 0,25 | 0,00 |
| CO2 | 0,00 | 0,04 | 0,01 | 0,06 | 0,00 |
| N2 | 0,00 | 0,00 | 0,00 | 0,00 | 0,00 |
| Massenstrom [kg/h] | 62,07 | 22,08 | 6,13 | 86,73 | 3,54 |

### Beispiel 2

### Abwasserextraktion mit Toluol

Abwasser aus dem Abscheidergefäß (Strom F) wird mit 90,54 kg/h bei einer Temperatur von 70°C in den oberen Teil einer gerührten Extraktionskolonne mit einem Durchmesser von 100 mm und einer Länge von 800 mm eingeleitet. Nach dem Gegenstromprinzip werden in den unteren Teil der Kolonne 16,9 kg/h Toluol (Strom G) mit 30°C zugeführt. Aufgrund des Dichteunterschieds läuft nach abgeschlossenem Extraktionsvorgang die Toluolphase (Strom H) am Kolonnenkopf in eine Vorlage über. Aus dem Kolonnensumpf tritt der gereinigte Abwasserstrom (Strom I) aus und kann der Dampfstrippung zugeführt werden.

**Tabelle 2**

| Komponente | Strom F | Strom G | Strom H | Strom I |
|---|---|---|---|---|
| | [kg/h] | [kg/h] | [kg/h] | [kg/h] |
| TOLUOL | 0,00 | 16,90 | 16,83 | 0,07 |
| MNT | 0,01 | 0,00 | 0,01 | 0,00 |
| DNT | 0,03 | 0,00 | 0,03 | 0,00 |
| NITROKRESOLE, PIKRINSÄURE, NITROBENZOESÄUREN | 0,05 | 0,00 | 0,04 | 0,01 |
| HNO3 | 0,44 | 0,00 | 0,00 | 0,44 |
| HNO2 | 0,09 | 0,00 | 0,00 | 0,09 |
| H2SO4 | 0,73 | 0,00 | 0,00 | 0,73 |
| H2O | 88,79 | 0,00 | 0,03 | 88,76 |
| NA2SO4 | 0,09 | 0,00 | 0,00 | 0,09 |
| NANO3 | 0,25 | 0,00 | 0,00 | 0,25 |
| CO2 | 0,06 | 0,00 | 0,00 | 0,06 |
| Massenstrom [kg/h] | 90,54 | 16,90 | 16,94 | 90,50 |

### Beispiel 3

### Toluolabtrennung mittels Dampfstrippung

Ein Abwasserstrom J aus dem Kolonnensumpf der Abwasserextraktion wird kontinuierlich mit 502,24 kg/h bei einer Temperatur von 60°C und Atmosphärendruck auf den Kopf einer Strippkolonne mit einem Durchmesser von 100 mm und einer Gesamthöhe von 1000 mm aufgegeben. Im Mittelteil ist die Kolonne mit einer Packung versehen. In den unteren Teil der Kolonne werden 20,1 kg/h Dampf mit 2,5 bar eingespeist. Aus dem Kolonnensumpf werden 501,17 kg/h gereinigtes Abwasser K mit einer Temperatur von 60-62°C entnommen. Über Kopf wird der Strippkolonne bei 200 bar Druck und 58-60°C ein Brüdenstrom L abgeführt. Mit einem Kondensator und einer der Vakuumpumpe vorgeschalteten Kühlfalle wird dieser Strom kondensiert.

**Tabelle 3**

| Komponente | Strom J | Strom K | Strom L |
|---|---|---|---|
| | [kg/h] | [kg/h] | [kg/h] |
| TOLUOL | 0,37 | 0,00 | 0,37 |
| HNO3 | 2,46 | 2,25 | 0,21 |
| HNO2 | 0,49 | 0,45 | 0,04 |
| H2SO4 | 4,05 | 4,05 | 0,00 |
| H2O | 492,95 | 492,52 | 20,53 |
| NA2SO4 | 0,52 | 0,52 | 0,00 |
| NANO3 | 1,39 | 1,39 | 0,00 |
| Massenstrom [kg/h] | 502,24 | 501,17 | 21,17 |

## Patentansprüche

1. Verfahren zur Aufarbeitung von wässrigen Abwässern, die bei der Nitrierung von Toluol zu Dinitrotoluol mit Nitriersäure anfallen, wobei die wässrigen Abwässer saures Waschwasser und alkalisches Waschwasser aus der Wäsche von Dinitrotoluol und Destillat aus der Schwefelsäureaufkonzentrierung enthalten, bei dem
a) die sauren und alkalischen Abwässer aus der Wäsche und das wässrige Destillat aus der Schwefelsäureaufkonzentrierung vereinigt werden, so dass sich ein pH-Wert von unter 5 einstellt, und anschließend die entstehende wässrige und organische Phase durch Phasentrennung getrennt werden, und
b) die wässrige Phase aus Schritt a) einer Extraktion zugeführt wird, wobei die in der wässrigen Phase enthaltenen organischen Komponenten mit Toluol extrahiert und die mit den organischen Komponenten angereicherte Toluolphase der Nitrierung von Toluol zugeführt wird.

2. Verfahren nach Anspruch 1, bei dem die Vereinigung der wässrigen Abwässer in einem Mischer mit Entgasung erfolgt.

3. Verfahren nach Anspruch 1 oder 2, bei dem die vereinigten wässrigen Phasen mit Mononitrotoluol vermischt werden.

4. Verfahren nach einem der Ansprüche 1 bis 3, bei dem die organische Phase aus Schritt a) wieder dem Nitrierprozess zugeführt wird.

5. Verfahren nach einem der Ansprüche 1 bis 4, bei dem die wässrige Phase aus Schritt a) mit Toluol in einem statischen Mischer vermischt und anschließend das Gemisch der Extraktion zugeführt wird.

6. Verfahren nach einem der Ansprüche 1 bis 4, bei dem wässrige Phase aus Schritt a) und Toluol bei der Extraktion im Gegenstrom geführt werden und in einem Gewichtsverhältnis von Toluol zu wässriger Phase von 33 : 100 bis 5 : 100 eingesetzt werden.

7. Verfahren nach einem der Ansprüche 1 bis 6, bei dem die Extraktion in Schritt b) in mehrstufigen, gerührten Extraktionskolonnen erfolgt.

8. Verfahren nach einem der Ansprüche 1 bis 7, bei dem die wässrige Phase aus Schritt b) durch Dampfstrippung von Toluol gereinigt wird, wobei ein Wasser / Toluol - Gemisch erhalten wird.

9. Verfahren nach Anspruch 8, bei dem für die Dampfstrippung eine Strippkolonne eingesetzt wird, die mit Packungen, Füllkörpern oder Böden ausgerüstet ist, wobei die wässrige Phase auf den Kopf der Strippkolonne aufgegeben wird und der Dampf in den unteren Bereich der Kolonne eingespeist wird.

10. Verfahren nach Anspruch 8, bei dem das erhaltene Wasser / toluoyl - Gemisch in die Extraktion in Schritt b) oder in den Nitrierprozess zurückgeführt wird.

## Claims

1. Process for working up aqueous waste water formed in the nitration of toluene to dinitrotoluene with nitrating acid, the aqueous waste water containing acidic wash water and alkaline wash water from the washing of dinitrotoluene and distillate from the concentration of sulfuric acid, in which process
a) the acidic and alkaline waste water from the washing and the aqueous distillate from the concentration of sulfuric acid are combined so that a pH value below 5 is established, and the resulting aqueous and organic phases are then separated by phase separation, and
b) the aqueous phase from step a) is fed to an extraction, wherein the organic components present in the aqueous phase are extracted with toluene and the toluene phase enriched with the organic components is fed to the nitration of toluene.

2. Process according to claim 1, in which combination of the aqueous waste water is carried out in a mixer with degassing.

3. Process according to claim 1 or 2, in which the combined aqueous phases are mixed with mononitrotoluene.

4. Process according to any one of claims 1 to 3, in which the organic phase from step a) is fed to the nitration process again.

5. Process according to any one of claims 1 to 4, in which the aqueous phase from step a) is mixed with toluene in a static mixer and the mixture is then fed to the extraction.

6. Process according to any one of claims 1 to 4, in which aqueous phase from step a) and toluene are fed countercurrently in the extraction and are used in a weight ratio of toluene to aqueous phase of from 33:100 to 5:100.

7. Process according to any one of claims 1 to 6, in which the extraction in step b) is carried out in multistage, stirred extraction columns.

8. Process according to any one of claims 1 to 7, in which toluene is removed from the aqueous phase from step b) by steam stripping, a water/toluene mixture being obtained.

9. Process according to claim 8, in which there is used for the steam stripping a stripping column which is equipped with packing, filling material or plates, the aqueous phase being introduced at the head of the stripping column and the steam being fed into the lower region of the column.

10. Process according to claim 8, in which the resulting water/toluene mixture is fed back into the extraction in step b) or into the nitrating process.

## Revendications

1. Procédé de traitement d'eaux usées aqueuses qui apparaissent lors de la nitration du toluène en dinitrotoluène avec le mélange sulfonitrique, où les eaux usées aqueuses contiennent une eau de lavage acide et une eau de lavage alcaline provenant du lavage du dinitrotoluène et un distillat provenant de la concentration de l'acide sulfurique où
a) les eaux usées acides et alcalines provenant du lavage et le distillat aqueux provenant de la concentration de l'acide sulfurique sont réunis de sorte qu'il s'établit un pH inférieur à 5, puis la phase aqueuse et la phase organique formées sont séparées par séparation des phases et
b) la phase aqueuse provenant de l'étape a) est envoyée à une extraction où les composants organiques contenus dans la phase aqueuse sont extraits avec du toluène et la phase de toluène enrichie avec les composants organiques est envoyée à la nitration du toluène.

2. Procédé selon la revendication 1 où la réunion des eaux usées aqueuses a lieu dans un mélangeur avec dégazage.

3. Procédé selon la revendication 1 ou 2 où les phases aqueuses réunies sont mélangées avec du mononitrotoluène.

4. Procédé selon l'une des revendications 1 à 3 où la phase organique provenant de l'étape a) est envoyée de nouveau au processus de nitration.

5. Procédé selon l'une des revendications 1 à 4 où la phase aqueuse provenant de l'étape a) est mélangée avec du toluène dans un mélangeur statique puis le mélange est envoyé à l'extraction.

6. Procédé selon l'une des revendications 1 à 4 où la phase aqueuse provenant de l'étape a) et le toluène sont conduits à contre-courant lors de l'extraction et sont utilisés dans un rapport en poids du toluène à la phase aqueuse de 33:100 à 5:100.

7. Procédé selon l'une des revendications 1 à 6 où l'extraction dans l'étape b) a lieu dans des colonnes d'extraction agitées à plusieurs étages.

8. Procédé selon l'une des revendications 1 à 7 où la phase aqueuse provenant de l'étape b) est purifiée par strippage à la vapeur du toluène, où un mélange eau/toluène est obtenu.

9. Procédé selon la revendication 8 où une colonne de strippage qui est équipée de garnissages , de corps de remplissage ou de plateaux est utilisée pour le strippage à la vapeur, où la phase aqueuse est appliquée à la tête de la colonne de strippage et la vapeur est introduite dans le domaine inférieur de la colonne.

10. Procédé selon la revendication 8 où le mélange eau/toluène obtenu est renvoyé dans l'extraction dans l'étape b) ou dans le processus de nitration.
